(19) Europäisches Patentamt
European Patent Office
Office européen des brevets

(11) Veröffentlichungsnummer : **0 173 226**
**B1**

(12) **EUROPÄISCHE PATENTSCHRIFT**

(45) Veröffentlichungstag der Patentschrift : **25.05.88**

(51) Int. Cl.⁴ : **C 07 C 69/67**, C 07 C 67/38

(21) Anmeldenummer : **85110428.1**

(22) Anmeldetag : **20.08.85**

(54) **Verfahren zur Herstellung von beta-Formylpropionsäure-tert.-butylester.**

(30) Priorität : **29.08.84 DE 3431642**

(43) Veröffentlichungstag der Anmeldung : **05.03.86 Patentblatt 86/10**

(45) Bekanntmachung des Hinweises auf die Patenterteilung : **25.05.88 Patentblatt 88/21**

(84) Benannte Vertragsstaaten : **CH DE FR GB IT LI**

(56) Entgegenhaltungen :
**US-A- 3 253 018**
**US-A- 3 337 603**

(73) Patentinhaber : **Ruhrchemie Aktiengesellschaft**
**Bruchstrasse 219**
**D-4200 Oberhausen 11 (DE)**

(72) Erfinder : **Bahrmann, Helmut, Dr. Dipl.-Chem.**
**Rohstrasse 48**
**D-4236 Hamminkeln (DE)**
Erfinder : **Cornils, Boy, Dr. Dipl.-Chem.**
**Friedrich-Ebert-Strasse 45**
**D-4220 Dinslaken (DE)**
Erfinder : **Konkol, Werner, Dr. Dipl.-Chem.**
**Lützowstrasse 40a**
**D-4200 Oberhausen 11 (DE)**
Erfinder : **Weber, Jürgen, Dr. Dipl.-Chem.**
**Bunsenstrasse 17**
**D-4200 Oberhausen 11 (DE)**
Erfinder : **Heim, Volker**
**Schützenstrasse 6**
**D-4200 Oberhausen 12 (DE)**

(74) Vertreter : **Reichelt, Karl-Heinz, Dr.**
**Ruhrchemie Aktiengesellschaft Abt. PLD Postfach 13 01 60**
**D-4200 Oberhausen 11 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von β-Formylpropionsäure-tert.-ester der Formel

$$OHC—CH_2—CH_2—COOC(CH_3)_3.$$

Es ist bekannt, β-Formylpropionsäure-ester aus Acrylsäureester durch Hydroformylierung, d. h. katalytische Umsetzung mit Kohlenmonoxid und Wasserstoff unter erhöhtem Druck herzustellen. Die Reaktion ergibt zwei isomere Verbindungen der allgemeinen Formel I (alpha-Formylpropionsäureester) und II (β-Formylpropionsäureester)

$$\begin{array}{c} CH_3 \\ | \\ OHC - CH - COOR \end{array} \qquad OHC - CH_2 - CH_2 - COOR$$

(I) (II)

deren Anteil im Hydroformylierungsprodukt von den Reaktionsbedingungen, darunter insbesondere von dem verwendeten Katalysator, abhängt.

Nach dem in C.A., Bd. 52 (1958), Spalte 14661 b beschriebenen Verfahren hydroformyliert man Acrylsäuremethylester in Gegenwart von Kobaltcarbonyl und Pyridin als Katalysator und Benzol als Lösungsmittel bei 120 °C und 200 atm. β-Formylpropionsäuremethylester bildet sich hierbei zu 85 %.

Ebenfalls mit Kobalt als Katalysator, jedoch in kontinuierlicher Arbeitsweise, erhält man nach der US-PS 33 37 603, Beispiel 6 aus Acrylsäuremethylester bei 130 °C und 200 atm. den β-Formylpropionsäuremethylester in einer Ausbeute von 80 %.

Durch Einsatz von Katalysatoren auf Basis von Kobaltkomplexverbindungen, die ditertiäre Phosphine als Liganden enthalten, kann der Anteil des β-Isomeren bei der Umsetzung von Acrylsäuremethylester mit Kohlenmonoxid und Wasserstoff im Reaktionsprodukt bis auf 86 % erhöht werden (C.A. 94, 1981, Referat Nr. 37138 t).

Weniger geeignet zur Herstellung von β-Formylpropionsäureestern als Kobaltkatalysatoren sind Rhodiumkatalysatoren zur Erzielung befriedigender Ausbeuten des β-Isomeren. Als nachteilig erweist sich hierbei, daß hohe Reaktionstemperaturen erforderlich sind. Wegen der thermischen Instabilität der Rhodiumkatalysatoren kommt es zu Rhodiumablagerungen im Reaktor und damit zum Verlust des wertvollen Metalls. Die thermisch stabileren Rhodium-Phosphin-Komplexkatalysatoren begünstigen die Bildung von alpha-Formylpropionsäureestern. Hinzu kommt, daß Rhodiumverbindungen auch in geringen Konzentrationen bei höheren Temperaturen die Hydrierung von Acrylsäureestern katalysieren, so daß Propionsäureester in erheblichem Umfang entstehen und der günstige Einfluß höherer Temperatur auf die Bildung des β-Isomeren nicht ausgenutzt werden kann. So hydroformyliert man z. B. nach dem Verfahren der NL-A-65 16193 Acrylsäuremethylester in Gegenwart von Rhodiumoxid als Katalyator bei 120 °C und 200 atm und erhält Formylpropionsäuremethylester (zwischen der alpha- und β-Form wird nicht unterschieden) in einer Ausbeute von 62 %.

In der japanischen Patentanmeldung 77/73.822 wird die Hydroformylierung verschiedener Acrylsäure- und Methacrylsäureester in Gegenwart von Rhodium-Phosphin-Komplexverbindungen als Katalysator beschrieben. Bei Einsatz von Acrylsäureethylester, Anwendung eines Druckes von 100 atm und einer Temperatur von 150 °C erhält man-Formylpropionsäureethylester in 45 % Ausbeute.

Die gezielte Herstellung von alpha-Formylpropionsäurealkylestern gelingt entsprechend dem in der DE-A1 26 43 205 offenbarten Verfahren bei 80 bis 140 °C und 180 bis 400 bar mit Hilfe von Rhodium-Komplexverbindungen, die als Liganden eine Phosphorverbindung der allgemeinen Formel $P[(O)_nR]_3$ enthalten.

Zusammenfassend ergibt sich, daß nach dem Stand der Technik β-Formylpropionsäureester nur in Ausbeuten, die deutlich unter 90 % liegen, hergestellt werden können. Eine zusätzliche Minderung der Ausbeute tritt bei der destillativen Reinigung der Produkte auf, die thermisch instabil sind und bei höherer Temperatur in schwerflüchtige Verbindungen übergehen.

Es bestand daher die Aufgabe, ein Verfahren zu entwickeln, das β-Formylpropionsäureester in hoher Ausbeute und so ausreichender Reinheit bereitstellt, daß eine zusätzliche destillative Reinigung des Reaktionsproduktes entbehrlich ist.

Diese Aufgabe wird gelöst durch ein Verfahren zur Herstellung von β-Formylpropionsäure-tert.-butylester. Es ist dadurch gekennzeichnet, daß Acrylsäure-tert.-butylester in Gegenwart von Kobaltkatalysatoren und in Anwesenheit eines inerten organischen Lösungsmittels bei 50 bis 150 bar ($5 \cdot 10^3$-$15 \cdot 10^3$ kPa) und 90 bis 130 °C mit Kohlenmonoxid und Wasserstoff umgesetzt, nach beendeter Reaktion der Kobaltkatalysator durch Komplexbildung in wasserunlösliche Form überführt und der β-Formylpropionsäure-tert.-butylester mit Wasser extrahiert wird.

Überraschenderweise erhält man nach dem erfindungsgemäßen Verfahren ein Produkt, das mindestens 98 % β-Formylpropionsäure-tert.-butylestr enthält. Dieses Ergebnis war, betrachtet man die unter

verschiedenen Reaktionsbedingungen und unter Einsatz unterschiedlicher Katalysatoren erzielten Reaktionsprodukte bei der Hydroformylierung anderer Ester als des tert.-Butylesters der Acrylsäure, nicht zu erwarten.

Die Hydroformylierung des Acrylsäure-tert.-butylesters erfolgt nach bekannten Techniken diskontinuierlich oder kontinuierlich. Hierbei sind Drucke von 50 bis 150 bar, vorzugsweise 70 bis 110 bar und Temperaturen von 90 bis 130 °C, vorzugsweise 110 bis 120 °C einzuhalten. Wasserstoff und Kohlenmonoxid werden zweckmäßigerweise in äquimolarem Gemisch eingesetzt, wenngleich ein geringer Überschuß eines der beiden Bestandteile nicht schadet.

Die Mitverwendung eines inerten organischen Lösungsmittels bei Durchführung der Hydroformylierung ist im Hinblick auf die schonende Aufarbeitung des Reaktionsgemisches geboten.

Als Lösungsmittel kommen aliphatische oder aromatische Kohlenwasserstoffe wie Cyclohexan, Isooctan, Benzol oder Toluol in Betracht. Besonders geeignet ist Toluol.

Um der Polymerisation des Acrylsäureesters entgegenzuwirken, empfiehlt sich ferner der Zusatz eines Polymerisationsinhibitors wie Hydrochinon in Mengen von 1 bis 10 mMol je Mol Ester.

Als Katalysator wird nach der neuen Arbeitsweise Kobalt in metallischer Form oder als Verbindung eingesetzt. Geeignete Verbindungen sind hierbei anorganische oder organische Salze des Kobalts wie Kobalthalogenide, Kobaltsulfat, Kobaltcarbonat, Kobaltacetat, Kobaltpropionat oder Kobalt-(2-ethylhexanoat). Aus Kobalt oder den Kobaltverbindungen bilden sich unter Einwirkung von Kohlenmonoxid und Wasserstoff Kobaltcarbonyl und/oder -carbonylhydride, die die eigentlichen Hydroformylierungskatalysatoren sind. Sie können im Reaktionsgemisch aus Kobaltmetall oder Kobaltsalze hergestellt werden. Es ist aber auch möglich, sie in einem eigenen Reaktor vorzuformen und dann dem Reaktionsgemisch zuzusetzen. Durch diese Maßnahme kann die Reaktionszeit beträchtlich herabgesetzt werden. Die Konzentration des Katalysators beträgt 0,2 bis 2 % Co, bezogen auf das Reaktionsgemisch.

Die Aufarbeitung des Reaktionsgemisches kann nach Abtrennung des Kobalts durch bekannte Verfahren zwar destillativ erfolgen. Im Hinblick auf die thermische Empfindlichkeit des Formylpropionsäure-tert.-butylesters und dem dadurch bedingten Verlust von Wertprodukt bei der Destillation empfiehlt es sich jedoch nicht, diesen Weg zu beschreiten. Sehr bewährt hat sich dagegen, das im Reaktionsprodukt als Carbonylverbindung gelöste Kobalt durch Komplexbildung in wasserunlösliche Form zu überführen und den β-Formylpropionsäureester aus dem Gemisch mit Wasser zu extrahieren. Als Komplexbildner für das Kobalt eignen sich insbesondere tertiäre Alkyl- oder Arylphosphine und -phosphite wie Triethylphosphin, Tri-n-butylphosphin, Tri-n-octylphosphin, Tri-n-laurylphosphin, Triphenylphosphin, Triethylphosphit, Tri-n-butylphosphit, Triphenylphosphit. Wegen seiner leichten Zugänglichkeit ist Triphenylphosphin der bevorzugte Komplexbildner. Um sicherzustellen, daß das gesamte Kobalt in wasserunlösliche Form überführt wird, muß das komplexbildende Reagenz, bezogen auf Kobalt, im Überschuß angewendet werden. Es empfiehlt sich, je gAtom Kobalt mindestens 1,2 Mol und insbesondere 2 bis 4 Mol Komplexbildner einzusetzen.

Die Abtrennung des Kobalts und die Isolierung des β-Formylpropionsäure-tert.-butylesters erfolgt in der Weise, daß dem entspannten Reaktionsgemisch eine ausreichende Menge Komplexbildner, gegebenenfalls gelöst in einem Lösungsmittel, vorzugsweise dem, das auch bei der Hydroformylierung Anwendung findet, zugesetzt wird. Darauf wird der β-Formylpropionsäureester mit Wasser extrahiert und die wäßrige Phase von der organischen Phase, die den Katalysator enthält, abgetrennt. Zur Gewinnung des reinen Esters wird das Wasser, zweckmäßig unter vermindertem Druck, abdestilliert.

β-Formylpropionsäure-tert.-butylester ist ein wichtiges Zwischenprodukt, das z. B. bei der Herstellung von Herbiziden umfangreich Verwendung findet. Durch Umesterung läßt sich der Butylester in andere Ester, durch Verseifung oder thermische Spaltung in die freie Säure überführen.

Im folgenden Beispiel wird die Erfindung näher beschrieben.

## Beispiel

In einem 5 l-Autoklaven werden 8,7 g $CoCO_3$ (1 % Co, bezogen auf eingesetztes Olefin) in 435 g Toluol suspendiert und zur Präformierung des Katalysators 1 Stunde bei 175 °C und 270 bar mit Kohlenmonoxid und Wasserstoff (Volumverhältnis 1 : 1) behandelt. Danach werden die Temperatur auf 115 °C und der Druck auf 100 bar abgesenkt und zum Autoklaveninhalt innerhalb 30 min 435 g Acrylsäure-tert.-butylester gepumpt. Nach weiteren 1 1/5 Stunden wird die Reaktion abgebrochen, der Autoklav abgekühlt und entspannt und das Reaktionsgemisch gaschromatographisch analysiert. Neben verschiedenen Komponenten in geringer Konzentration enthält das Reaktionsprodukt 54,2 % β-Formylpropionsäure-tert.-butylester und 1,1 % alpha-Formylpropionsäure-tert.-butylester, entsprechend einem Verhältnis alpha-Verbindung/β-Verbindung von 2 : 98, sowie 41 % Toluol.

## Vergleichsbeispiel

Das vorstehende Beispiel wird identisch wiederholt mit dem Unterschied, daß man anstelle von Acrylsäure-tert.-butylester Acrylsäuremethylester einsetzt. Die gaschromatographische Analyse des Reaktionsproduktes ergibt neben verschiedenen Komponenten in geringer Konzentration einen Gehalt von 33,4 % β-Formylpropionsäuremethylester und 4,7 % alpha-Formylpropionsäuremethylester, entsprechend einem Verhältnis alpha-Verbindung/β-Verbindung von 12 : 86 sowie 48,2 % Toluol. Ähnliche Ergebnisse erhält man bei Einsatz von Acrylsäureethylester.

Das Vergleichsbeispiel macht deutlich, daß durch Auswahl von Acrylsäure-tert.-butylester als Einsatzmaterial für die Hydroformylierung überraschenderweise der β-Formylpropionsäureester mit höherer Selektivität erhalten wird als bei der Hydroformylierung anderer Ester der Acrylsäure.

**Patentansprüche**

1. Verfahren zur Herstellung β-Formylpropionsäure-tert.-butylester, dadurch gekennzeichnet, daß Acrylsäure-tert.-butylester in Gegenwart von Kobaltkatalysatoren und in Anwesenheit eines inerten organischen Lösungsmittels bei 50 bis 150 bar ($5 \cdot 10^3$-$15 \cdot 10^3$ kPa) und 90 bis 130 °C mit Kohlenmonoxid und Wasserstoff umgesetzt, nach beendeter Reaktion der Kobaltkatalysator durch Komplexbildung in wasserunlöslicher Form überführt und der β-Formylpropionsäure-tert.-butylester mit Wasser extrahiert wird.

2. Verfahren nach Anspruch 1, dadurch gekennzeichnet, daß die Umsetzung bei 70 bis 110 bar und Temperaturen von 110 bis 120 °C durchgeführt wird.

3. Verfahren nach Anspruch 1 und 2, dadurch gekennzeichnet, daß die Konzentration des Katalysators 0,2 bis 2 Gew.% Co, bezogen auf das Reaktionsgemisch, beträgt.

4. Verfahren nach Anspruch 1 bis 3, dadurch gekennzeichnet, daß das komplexbildende Reagenz in einer Menge von mindestens 1,5 Mol, insbesondere 2 Mol bis 4 Mol je gAtom Kobalt eingesetzt wird.

**Claims**

1. A process for the preparation of β-formylpropionic acid tertiary butylester, characterised in that acrylic acid tertiary butylester is reacted with carbon monoxide and hydrogen in the presence of cobalt catalysts and in the presence of an inert organic solvent at 50 to 150 bar ($5 \times 10^3$-$15 \times 10^3$ kPa) and 90 to 130 °C, after the reaction has been completed the cobalt catalyst is converted into a water insoluble form and the β-formylpropionic acid tertiary butylester is extracted with water.

2. A process according to claim 1, characterised in that the reaction is performed at 70 at 110 bar and temperatures of 110 to 120 °C.

3. A process according to claims 1 and 2, characterised in that the concentration of the catalyst is 0.2 to 2 wt.% Co, related to the reaction mixture.

4. A process according to claims 1 to 3, characterised in that the complex-forming reagent is used in an amount of at least 1.5 mol, in particular 2 to 4 mol per g atom cobalt.

**Revendications**

1. Procédé pour la fabrication de β-formylpropionate de tert-butyle, caractérisé en ce que l'on fait réagir l'acrylate de tert-butyle avec le monoxyde de carbone et l'hydrogène en présence de catalyseurs au cobalt et en présence d'un solvant organique inerte sous 50-150 bars ($5 \cdot 10^3$-$15 \cdot 10^3$ kPa) et à 90-130 °C, on transforme après la fin de la réaction le catalyseur au cobalt en une forme insoluble dans l'eau par formation de complexe et on extrait par l'eau le β-formylpropionate de tert-butyle.

2. Procédé selon la revendication 1, caractérisé en ce que la réaction est mise en œuvre sous 70-110 bars et à des températures de 110-120 °C.

3. Procédé selon les revendications 1 et 2, caractérisé en ce que la concentration du catalyseur est de 0,2 à 2 % en poids de Co par rapport au mélange de réaction.

4. Procédé selon les revendications 1 à 3, caractérisé en ce que le réactif complexant est utilisé en quantité d'au moins 1,5 mole par atome-gramme de cobalt, en particulier de 2 à 4 moles par atome-gramme.